(19) Europäisches Patentamt
European Patent Office
Office européen des brevets

(11) **EP 3 207 017 B1**

(12) **EUROPÄISCHE PATENTSCHRIFT**

(45) Veröffentlichungstag und Bekanntmachung des Hinweises auf die Patenterteilung:
**26.09.2018 Patentblatt 2018/39**

(21) Anmeldenummer: **15778337.4**

(22) Anmeldetag: **12.10.2015**

(51) Int Cl.:
*C07C 37/84* (2006.01)       *C07C 39/06* (2006.01)
*C07C 37/14* (2006.01)       *C07C 37/74* (2006.01)

(86) Internationale Anmeldenummer:
**PCT/EP2015/073538**

(87) Internationale Veröffentlichungsnummer:
**WO 2016/058978 (21.04.2016 Gazette 2016/16)**

(54) **VERBESSERTES VERFAHREN ZUR HERSTELLUNG VON PARA-THYMOL**

IMPROVED METHOD FOR PRODUCING PARA-THYMOL

PROCÉDÉ AMÉLIORÉ DESTINÉ À FABRICATION DU PARA-THYMOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.10.2014 EP 14188640**

(43) Veröffentlichungstag der Anmeldung:
**23.08.2017 Patentblatt 2017/34**

(73) Patentinhaber: **Lanxess Deutschland GmbH 50569 Köln (DE)**

(72) Erfinder:
• **BÖGER, Uwe**
  **51375 Leverkusen (DE)**
• **HEUER, Lutz**
  **41542 Dormagen (DE)**
• **ZIRNGIEBL, Eberhard**
  **51061 Köln (DE)**
• **HENSELER, Alexander**
  **50937 Köln (DE)**
• **FARKAS, Eszter**
  **40723 Hilden (DE)**

(56) Entgegenhaltungen:
**EP-A1- 2 524 906**

• **H. Becker et al: "Organikum", 1996, Johann Ambrosius Barth Verlag, Heildelberg - Leipzig, XP002735608, ISBN: 3-335-00492-2 Seiten 34-36, Seite 34 - Seite 36**

**Beschreibung**

Die vorliegende Erfindung betrifft ein verbessertes Verfahren zur Herstellung von 4-Isopropyl-3-methyl-phenol (p-Thymol) aus Destillationsrückständen der Thymolherstellung.

4-Isopropyl-3-methyl-phenol wird beispielsweise als antibakterielles und mikrobizides Mittel in Kosmetika und Mundwässern mit Anti-Plaque-Wirkung sowie Fuß- und Haarpflegemitteln mit guter Hautverträglichkeit eingesetzt. Die Herstellung von 4-Isopropyl-3-methyl-phenol ist prinzipiell bekannt.

So beschreibt US 3,331,879 die Umsetzung von meta-Kresol (m-Kresol, 3-Methylphenol) mit Propen an einem Zirkoniumkatalysator, wobei im Wesentlichen Thymol (2-Isopropyl-5-methyl-phenol), aber auch viele aromatische Nebenprodukte entstehen. Als ein Nebenprodukt wurde 4-Isopropyl-3-methyl-phenol mit einem Gehalt von 2 % in der Reaktionsmischung bzw. 4,4 % nach einer ersten Destillation identifiziert. Die Isolierung des 4-Isopropyl-3-methyl-phenols wird nicht beschrieben.

DE 2139622 OS beschreibt den Anfall von bis zu 19,5 % 4-Isopropyl-3-methyl-phenol bei der Umsetzung von m-Kresol mit Propen an einem sauren Zinkkatalysator. Auch hier wird die Isolierung des 4-Isopropyl-3-methyl-phenols nicht beschrieben.

DE 2528303 OS beschreibt den Anfall von ca. 2 % 4-Isopropyl-3-methyl-phenol bei der Umsetzung von meta-Kresol mit Propen an einem basischen Aluminiumoxid-Katalysator. Isolierung des 4-Isopropyl-3-methyl-phenols aus den vielfältigen Nebenprodukten wird nicht beschrieben.

Weiterhin ist aus US 2,603,662 bekannt 4-Isopropyl-3-methyl-phenol über einen aufwändigen Prozess als Nebenprodukt bei der Umsetzung von meta-Kresol mit Isobuten zu gewinnen.

Allen vorgenannten Verfahren ist gemeinsam, dass 4-Isopropyl-3-methyl-phenol als Nebenkomponente bei der Alkylierung von m-Kresol mit so vielen anderen Nebenkomponenten anfällt, dass eine Gewinnung entweder nicht vorgenommen wurde oder außerordentlich umständlich erfolgt.

Aus DE 102007035515 A ist ein Verfahren bekannt, bei dem Thymol und nicht-umgesetztes meta-Kresol zunächst destillativ aus einer Reaktionsmischung aus der Thymolherstellung weitgehend abgetrennt werden und der dabei verbleibende Rückstand destilliert wird, um schwerst- oder nichtflüchtige Substanzen abzutrennen, und das so erhaltene Destillat nach Zusatz von bis zu 5 Gew.-% Wasser kristallisiert wird oder der verbleibende Rückstand nach Zusatz von bis zu 5 Gew.-% Wasser kristallisiert wird und der kristallisierte Rückstand durch Destillation von schwerst- oder nichtflüchtigen Substanzen getrennt wird.

EP 2524906 A1 offenbart ein Verfahren, bei dem Thymol und nicht umgesetztes meta-Kresol zunächst destillativ aus einer Reaktionsmischung aus der Thymolherstellung weitgehend abgetrennt werden. In einem anschließenden Kristallisationsverfahren wird p-Thymol mit einem Gehalt von 82 bis 87 Gew.% nach langsamen Abkühlen auf Raumtemperatur aus dem Destillationsrückstand auskristallisiert und isoliert. Das p-Thymol-Rohprodukt wird in einem weiteren Schritt mit Methylcylohexan und Aktivkohle umkristallisiert. Nach Filtration und Trocknung erhält man weiße Kristalle mit einer Reinheit von 99,8 Gew.% p-Thymol.

Es bestand daher das Bedürfnis, ein verbessertes Verfahren bereitzustellen, mit dem 4-Isopropyl-3-methyl-phenol in effizienter Weise und hoher Reinheit kostengünstig und ohne Umkristallisation oder Waschen mit organischen Lösungsmitteln gewonnen werden kann.

Es wurde nun ein Verfahren zur Herstellung von 4-Isopropyl-3-methyl-phenol aus Reaktionsmischungen gefunden, die durch Umsetzung von meta-Kresol mit Propen in Gegenwart eines Katalysators erhältlich sind, dadurch gekennzeichnet, dass zumindest

a) Thymol und nicht-umgesetztes meta-Kresol destillativ aus der Reaktionsmischung weitgehend abgetrennt werden, wobei ein Destillationsrückstand gewonnen wird, und

b) aus dem gemäß Schritt a) verbleibenden Destillationsrückstand eine Suspension enthaltend kristallines 4-Isopropyl-3-methyl-phenol und eine flüssige Phase durch Kristallisation erhalten wird, und

c) aus der gemäß Schritt b) erhaltenen Suspension kristallines 4-Isopropyl-3-methylphenol von der flüssigen Phase durch Einwirkung von Zentrifugalkräften, abgetrennt wird.

Bevorzugt umfasst das erfindungsgemäße Verfahren, besonders bevorzugt in Schritt c), keinen Waschschritt. Unter Waschschritt wird erfindungsgemäß eine Behandlung des kristallinen 4-Isopropyl-3-methyl-phenols mit Lösungsmitteln verstanden, bei dem das kristalline 4-Isopropyl-3-methyl-phenol selbst nicht gelöst, sondern nur daran anhaftende Verunreinigungen gelöst und damit vom kristallinen 4-Isopropyl-3-methyl-phenol abgetrennt werden.

Mit dem erfindungsgemäßen Verfahren kann eine sonst erforderliche aufwändige Reinigung des 4-Isopropyl-3-methyl-phenols durch Umkristallisation aus Lösungsmitteln zum Erhalt von hohen Reinheitsgraden vermieden werden.

Das gemäß Schritt c) erhaltene kristalline 4-Isopropyl-3-methyl-phenol weist typischerweise einen Gehalt von mehr als 90 Gew.%, bevorzugt von 91 bis 99 Gew.%, besonders bevorzugt von 92 bis 99 Gew.%, ganz besonders bevorzugt von 92 bis 96 Gew.% 4-Isopropyl-3-methyl-phenol auf.

Der Rest zu 100 Gew.% umfasst typischerweise andere Produkte, wie insbesondere weitere Isomere von Isopropylmethylphenol.

In einer Ausführungsform umfasst das erfindungsgemäße Verfahren zusätzlich den Schritt

d) Umkristallisation des aus Schritt c) erhaltenen kristallinen 4-Isopropyl-3-methylphenols.

Bevorzugt wird die Umkristallisation mit Methylcyclohexan, besonders bevorzugt in Gegenwart von Aktivkohle, durchgeführt.

Mit diesem Schritt kann die Reinheit des kristallinen 4-Isopropyl-3-methyl-phenols je nach Gehalt, der nach Schritt c) erhalten wird auf mehr als 96, bevorzugt von 97 bis 100 Gew.% erhöht werden.

Der Rahmen der Erfindung umfasst neben den genannten Bereichen und Vorzugsbereichen von Formeln und Parametern auch beliebige Kombinationen davon, selbst wenn sie aus praktischen Gründen nachstehend nicht vollständig explizit aufgeführt sind.

Bei der Alkylierung von meta-Kresol mit Propen in Gegenwart eines Katalysators, die in einer dem Fachmann bekannten Art und Weise durchgeführt werden kann (siehe z.B. DE 3824284 OS oder DE 2528303 OS) entsteht typischerweise ein Reaktionsgemisch, das neben einer Hauptmenge an Thymol auch etwa 1 bis 3 Gew.-% 4-Isopropyl-3-methylphenol enthält.

In einem Schritt a) des erfindungsgemäßen Verfahrens werden Thymol und nicht-umgesetztes meta-Kresol aus der Reaktionsmischung destillativ weitgehend abgetrennt. Der Begriff weitgehend bedeutet dabei, dass der verbleibende Rückstand einen Anteil an Thymol und meta-Kresol von zusammengenommen insgesamt 80 Gew.% oder weniger, bevorzugt 55 Gew.% oder weniger und besonders bevorzugt 30 Gew.% oder weniger aufweist.

Die Destillation kann dabei in an sich bekannter Weise beispielsweise diskontinuierlich oder kontinuierlich durchgeführt werden, wobei eine kontinuierliche Destillation unter gegenüber Normaldruck vermindertem Druck bevorzugt ist, der beispielsweise 1 bis 950 hPa, bevorzugt von 50 bis 950 hPa und besonders bevorzugt von 50 bis 150 hPa beträgt.

Die Temperatur bei der Destillation beträgt am Kolonnenkopf beispielsweise von 100 bis 225°C und bevorzugt von 140 bis 155°C, die Sumpftemperatur beträgt beispielsweise von 120 bis 260°C und bevorzugt von 170 bis 190°C, wobei dem Fachmann klar ist, dass die Temperaturen bei der Destillation am Kolonnenkopf und im Sumpf miteinander sowie mit dem Destillationsdruck korrelieren. Geeignete Destillationsbedingungen sind für den Fachmann ohne Weiteres ermittelbar.

Vorzugsweise erfolgt die Destillation mit Hilfe eines Kurzwegverdampfers, einer Kolonne mit oder ohne Einbauten oder durch einen Fallfilmverdampfer oder auch einen Dünnschichtverdampfer. Für die Destillation ist eine theoretische Trennstufe ausreichend. Die Anwendung von mehr als einer Trennstufe ist natürlich möglich, aber nicht erforderlich.

Typischerweise enthält der verbleibende Rückstand neben 4-Isopropyl-3-methylphenol 20 bis 30 weitere Nebenkomponenten niedermolekularer Struktur sowie polymere Nebenkomponenten. Nach der Destillation beträgt der Gehalt an 4-Isopropyl-3-methylphenol im verbleibenden, üblicherweise schwarz gefärbten Rückstand typischerweise 10 bis 30 Gew.-%.

Der gemäß Schritt a) verbleibende Destillationsrückstand weist vorzugsweise eine Temperatur von 120 bis 180°C und besonders bevorzugt von 120 bis 140°C auf.

Gemäß Schritt b) wird aus dem gemäß Schritt a) verbleibenden Destillationsrückstand 4-Isopropyl-3-methylphenol kristallisiert, wobei eine Suspension enthaltend kristallines 4-Isopropyl-3-methyl-phenol und eine flüssige Phase erhalten wird.

Schritt b) kann bevorzugt durch Abkühlen des Destillationsrückstandes zum Beispiel auf Temperaturen zwischen -20° und 30°C, bevorzugt auf 0 bis 30°C und besonders bevorzugt auf 3 bis 25°C oder Umgebungstemperatur erfolgen.

In einer anderen Ausführungsform gelingt die Kristallisation durch Abkühlen des gemäß Schritt a) verbleibenden Destillationsrückstandes, wobei die Temperaturdifferenz zwischen Beginn und Ende des Kristallisationsvorganges beim Abkühlen beispielsweise 30 K oder mehr, vorzugsweise 40 K oder mehr, bevorzugt von 40 bis 100 K beträgt.

In einer Ausführungsform des erfindungsgemäßen Verfahrens kann die Kristallisation durch Animpfen mit kristallinem 4-Isopropyl-3-methyl-phenols induziert oder beschleunigt werden.

Die Zeitdauer der Kristallisation beträgt typischerweise 5 bis 100 Stunden, vorzugsweise 5 bis 20 Stunden und besonders bevorzugt 5 bis 12 Stunden.

In Schritt b), d.h. während der Kristallisation, wird mechanisch durchmischt oder nicht, wobei die mechanische Durchmischung bevorzugt ist. Dies hat den Vorteil, dass dabei kleinere Kristalle entstehen, die im folgenden Schritt c) noch effektiver von der flüssigen Phase abgetrennt werden können.

Bei der mechanischen Durchmischung kann jede dem Fachmann dafür bekannte Vorrichtung eingesetzt werden. Beispielsweise umfassen Rühreinrichtungen Axialrührer, Radialrührer und Tangentialrührer. Rühreinrichtungen weisen zumindest ein Rührorgan, beispielsweise Propeller, Wendel oder Blätter, auf, die eine Strömung der flüssigen Phase erzeugen. Rühreinrichtungen weisen außerdem typischerweise einen Antrieb, z.B. einen Motor, und eine Verbindung zwischen Rührorgan und Antrieb, z.B. eine Welle oder eine magnetische Kopplung auf. Je nach Rührertyp werden Strömungen in radialer Richtung, d.h. rechtwinklig zur Rührachse, oder in axialer Richtung, d.h. parallel zur Rührachse, oder Mischungen davon erzeugt. Beispielsweise erzeugen Blattrührer bevorzugt radiale Strömungen, Schrägblattrührer und Propellerrührer axiale Strömungen. Axiale Strömungen können nach oben oder nach unten gerichtet sein.

Die nach Schritt b) erhaltene Suspension enthaltend kristallines 4-Isopropyl-3-methylphenol und eine flüssige Phase weist bevorzugt eine Temperatur von maximal 30°C, bevorzugt maximal 25°C, besonders bevorzugt zwischen -10° und 25°C, auf.

Die nach Schritt b) erhaltene Suspension weist eine Viskosität zwischen 5°C und 30°C in einem Bereich von 20 bis 250 MPas und weist eine Dichte zwischen 5 und 30°C von 1 bis 0,9 g/cm$^3$, bevorzugt von 0,98 bis 0,95 g/cm$^3$ auf. Die Viskosität wurde über ein Kapillarviskosimeter, die Dichte über ein Helium-Vergleichs-Pyknometer bestimmt.

In der nach Schritt b) erhaltenen Suspension liegt das 4-Isopropyl-3-methyl-phenol überwiegend kristallin mit Abmessungen in der Größenordnung von bis zu 800 μm, bevorzugt von 300 bis 500 μm, bei einer Konzentration von etwa 50 bis 200 g/l, bevorzugt von 70 bis 180 g/l, vor. In einer bevorzugten Ausführungsform liegen 80 oder mehr Gew.% des in der Suspension enthaltenen 4-Isopropyl-3-methyl-phenol als Kristall mit Abmessungen in der Größenordnung von bis zu 800 μm, bevorzugt von 300 bis 500 μm, bei einer Konzentration von etwa 50 bis 150 g/l, vor. Die Kristalle weisen überwiegend eine kubische Form auf. Unter kubischer Kristallform wird erfindungsgemäß ein Kristallsystem definiert, das sich auf ein dreidimensionales Achsenkreuz beziehen lässt, bei dem die Winkel der Achsen zueinander 90° betragen, und bei dem alle Kanten des Kristalls mit einer Toleranz von 10% gleich lang sind.

Ein Teil der flüssigen Phase der nach Schritt b) erhaltenen Suspension kann durch einfache Trennoperationen wie Sedimentation und Dekantieren, Filtration oder andere dem Fachmann bekannte einfache fest-flüssig Trennoperationen, die über die Schwerkraft funktionieren, abgetrennt werden, oder eben nicht. Allerdings reichen diese einfachen Trennoperationen nicht aus, um das 4-Isopropyl-3-methyl-phenol in der gewünschten, erfindungsgemäßen Qualität zu erhalten.

Im nachfolgenden Schritt c) wird aus der gemäß Schritt b) erhaltenen Suspension kristallines 4-Isopropyl-3-methyl-phenol von der flüssigen Phase durch Einwirkung von Zentrifugalkräften abgetrennt.

Unter Zentrifugalkraft wird erfindungsgemäß eine Trägheitskraft definiert, die radial von der Rotationsachse eines Körpers nach außen gerichtet ist. Sie wird durch die Trägheit des Körpers verursacht. Die Zentrifugalkraft ergibt sich aus der Zentrifugalbeschleunigung durch Multiplikation mit der Masse.

In einer Ausführungsform, in der Schritt c) unter Einwirkung von Zentrifugalkräften durchgeführt wird, erfolgt die Abtrennung des 4-Isopropyl-3-methyl-phenols von der flüssigen Phase in Zentrifugen. Zentrifugen teilen sich in die Gruppen Sedimentations- und Filtrationszentrifugen ein. Beide Typen unterteilen sich weiterhin in kontinuierliche und diskontinuierliche Zentrifugen. Zentrifugen weisen typischerweise rotierende Elemente, beispielsweise Trommeln, wie bei Trommelzentrifugen, oder an Drehachsen befestigte Becher, wie bei Filterbecherzentrifugen, auf, in die ein Fest-Flüssiggemisch eingebracht wird. Durch die durch Rotation entstehende Zentrifugalkraft wird das Fest-Flüssiggemisch gegen die Außenwand des rotierenden Elements gepresst. Im Fall von Filtrationszentrifugen mit Trommel als rotierendem Element befindet sich eine Filterschicht am äußeren Trommelmantel, die den Feststoff zurückhält, während das flüssige Filtrat durch die in der Filterschicht enthaltenen Poren oder Löcher nach außen abgeschleudert wird. Als Filterschicht wird bevorzugt eine textile Filterschicht eingesetzt. Die Trommel kann, wie bei Vertikalzentrifugen, vertikal oder, wie bei Horizontalzentrifugen oder Schälzentrifugen, horizontal gelagert sein. Horizontal- oder Vertikalzentrifugen gehören zu den diskontinuierlichen Zentrifugen, da der sich an der Trommelwand ablagernde Filterkuchen diskontinuierlich entnommen werden muss. Zu den kontinuierlichen Filterzentrifugen gehören zum Beispiel Schubzentrifugen, Gleitzentrifugen oder Siebschneckenzentrifugen, in denen der abgetrennte Feststoff kontinuierlich zu einem Auslass und dann aus dem System heraus gefördert wird, während das abzutrennende Gemisch kontinuierlich über einen Einlass in das System hinein gefördert wird. In Sedimentationszentrifugen setzt sich der in der Flüssigkeit enthaltene Feststoff unter der Wirkung eines Kraftfeldes (in der Regel Zentrifugalkraft) ab. Das sich bildende Sediment (Dickschlamm) sowie die geklärte Flüssigkeit (Filtrat) können sowohl kontinuierlich als auch diskontinuierlich abgezogen werden.

Schälzentrifungen stellen einen besonderen Typ der Horizontalzentrifugen dar. Nach Beendigung eines Zentrifugenzyklus wird der Feststoff mit einem Schälwerkzeug, beispielsweise einem Schälmesser, von dem Filterkuchen bei rotierender Trommel ausgeschält und aus dem System herausgefördert, beispielsweise über eine Austragsrutsche oder eine Austragsschnecke.

Die Temperatur der nach Schritt b) erhaltenen Suspension wird während Schritt c) bevorzugt bei maximal 30°C, besonders bevorzugt bei maximal 25°C, ganz besonders bevorzugt zwischen -10° und 25°C gehalten.

Schritt c) des erfindungsgemäßen Verfahrens wird beispielsweise in einer Zentrifuge, bevorzugt in einer Filterzentrifuge, weiter bevorzugt in einer diskontinuierlichen Filterzentrifuge, oder bevorzugt in einer Horizontalzentrifuge, besonders bevorzugt in einer Schälzentrifuge, durchgeführt. In einer Ausführungsform, in der Schritt c) des erfindungsgemäßen Verfahrens in einer der oben genannten Zentrifuge durchgeführt wird, weisen die Zentrifugen Trommeldurchmesser von 0,5 bis 2 m, bevorzugt von 0,8 bis 1,5 m, auf.

In einer Ausführungsform, in der Schritt c) des erfindungsgemäßen Verfahrens erfolgt die Anwendung von Zentrifugalkräften derart, dass die gemäß Schritt b) erhaltene Suspension einer relativen Zentrifugalbeschleunigung (RZB) von 100 bis 1000 g, bevorzugt von 200 bis 900 g, besonders bevorzugt von 600 bis 800 g, ausgesetzt wird. Entsprechende Vorrichtungen sind dem Fachmann wie oben beschrieben hinlänglich bekannt.

Die relative Zentrifugalbeschleunigung ist hierbei definiert als Vielfaches der mittleren Erdbeschleunigung g. Diese kann z.B. aus dem Radius der Zentrifugentrommel und der Umdrehungszahl berechnet werden nach der Formel:

$$RZB = \frac{4\pi^2}{g} r\, n^2$$

wobei g die mittlere Erdbeschleunigung (9,80665 m/s$^2$), r der Radius der Trommel in Meter und n die Drehzahl der Trommel in s$^{-1}$ ist.

Die Anwendung der vorgenannten Zentrifugalkräfte erfolgt vorzugsweise für eine Dauer von 5 bis 30 Minuten, bevorzugt von 10 bis 25 Minuten und besonders bevorzugt von 12 bis 20 Minuten.

Vorzugsweise werden dabei 80 Gew.% oder mehr der flüssigen Phase, bevorzugt 90 Gew.-% oder mehr der flüssigen Phase vom kristallinen 4-Isopropyl-3-methyl-phenol abgetrennt.

Der besondere Vorteil der Erfindung ist darin zu sehen, dass 4-Isopropyl-3-methyl-phenol als Nebenkomponente bei der Thymol-Herstellung trotz Vorliegen sehr vieler weiterer Nebenkomponenten effizient und in außerordentlich hoher Reinheit gewonnen werden kann.

### Beispiele

### Beispiel 1:

21,1 kg eines 60°C warmen, schwarzen Destillationsrückstandes aus der Herstellung von Thymol durch Umsetzung von m-Kresol mit Propen in Gegenwart eines Katalysators, der Destillationsrückstand enthaltend 21,5 Gew.-% Thymol, 7,0 Gew.-% 3-Isopropyl-5-methyl-phenol, 25,0 Gew.-% 4-Isopropyl-3-methyl-phenol, 21,5 Gew.-% 2,6-Diisopropyl-3-methyl-phenol, 21,0 Gew.-% 2,4-Diisopropyl-5-methyl-phenol sowie etwa 22 verschiedene in Summe ca. 4 Gew.-% sonstiger alkylierter Kresole wurden in einem 25-l fassenden Reaktor innerhalb von 16 Stunden unter Rühren langsam auf Raumtemperatur abgekühlt, wobei sich eine Suspension enthaltend kristallines 4-Isopropyl-3-methyl-phenol und eine flüssige Phase bildete. Das ausgefallene 4-Isopropyl-3-methyl-phenol wurde abfiltriert. Es wurden 5,26 kg 4-Isopropyl-3-methyl-phenol mit einer Reinheit von 83,4 Gew.-% erhalten, was einer Ausbeute von 82,3 Gew.-% des im Destillationsrückstand vorhandenen 4-Isopropyl-3-methyl-phenol entsprach.

### Beispiel 2:

Verwendet wurde eine Schälzentrifuge mit folgenden Parametern:

| | |
|---|---|
| Durchmesser der Trommel: | 1 m |
| Filterfläche: | 1,57 m$^2$ |
| Füllvolumen: | 172 l |
| Bordhöhe: | 0,125 m |
| Länge der Trommel: | 0,5 m |

Eingesetzt wurde eine Suspension mit folgenden Parametern:

| | |
|---|---|
| Temperatur: | 10°C |
| Dichte: | 0,97 g/cm$^3$ |
| Viskosität: | 112,5 mPas |
| Konzentration 4-Isopropyl-3-methyl-phenol: | 150 g/l |

Das erfindungsgemäße Verfahren wurde mit folgenden Parametern durchgeführt:

| | |
|---|---|
| relative Zentrifugalbeschleunigung: | 700 g |
| Zulaufvolumen Suspension: | 120 l/min |
| Zulaufdauer Suspension: | 180 s |

Nach der Beendigung des Zulaufs der Suspension wurde die relative Zentrifugalbeschleunigung der Trommel für 900 s auf 1000 g erhöht. Danach wurde die relative Zentrifugalbeschleunigung der Trommel auf 45 g erniedrigt und das Produkt mit einem Schälmesser ausgetragen. Es wurden 63 kg kristallines 4-Isopropyl-3-methyl-phenol erhalten, das einen Gehalt an 4-lsopropyl-3-methyl-phenol von 96,1 Gew.-% aufwies.

**Beispiel 3 (Umkristallisation):**

200 g 4-lsopropyl-3-methyl-phenol aus Beispiel 2 werden mit 580 g Methylcyclohexan und 5 g Aktivkohle versetzt und 10 Minuten unter Rückfluss erhitzt. Die Aktivkohle wird heiß abfiltriert und das Filtrat unter Rühren auf 25°C mit einer Kühlrate von 0,5 K/Minute abgekühlt.

Das auskristallisierte 4-lsopropyl-3-methyl-phenol wird abfiltriert und die Kristalle auf der Nutsche einmal mit 100 g Methylcyclohexan nachwaschen. Der abfiltrierte Feststoff wird bei 40°C/50 mbar im Vakuumtrockenschrank getrocknet.

| | |
|---|---|
| Ausbeute: | 169 g 4-lsopropyl-3-methyl-phenol |
| Gehalt: | 99,9 Gew.% |
| Schmelzpunkt: | 11,5-112,5°C |

**Patentansprüche**

1. Verfahren zur Herstellung von 4-Isopropyl-3-methyl-phenol aus Reaktionsmischungen, die durch Umsetzung von meta-Kresol mit Propen in Gegenwart eines Katalysators erhältlich sind, **dadurch gekennzeichnet, dass** zumindest

   a) Thymol und nicht-umgesetztes meta-Kresol destillativ aus der Reaktionsmischung weitgehend abgetrennt werden, wobei ein Destillationsrückstand gewonnen wird, und
   b) aus dem gemäß Schritt a) verbleibenden Destillationsrückstand eine Suspension enthaltend kristallines 4-lsopropyl-3-methyl-phenol und eine flüssige Phase durch Kristallisation erhalten wird, und
   c) aus der gemäß Schritt b) erhaltenen Suspension kristallines 4-Isopropyl-3-methyl-phenol von der flüssigen Phase durch Einwirkung von Zentrifugalkräften, abgetrennt wird.

2. Verfahren nach Anspruch 1, umfassend weiterhin den Schritt

   d) Umkristallisation des aus Schritt c) erhaltenen kristallinen 4-Isopropyl-3-methyl-phenols.

3. Verfahren nach Anspruch 1 oder 2, **dadurch gekennzeichnet, dass** Schritt c) durch Einwirkung von Zentrifugalkräften in einer Zentrifuge, bevorzugt in einer Filterzentrifuge, weiter bevorzugt in einer diskontinuierlichen Filterzentrifuge, oder bevorzugt in einer Horizontalzentrifuge, besonders bevorzugt in einer Schälzentrifuge, durchgeführt wird.

4. Verfahren nach einem der Ansprüche 1 bis 3, **dadurch gekennzeichnet, dass** es keinen Waschschritt umfasst.

5. Verfahren nach einem der Ansprüche 1 bis 4, **dadurch gekennzeichnet, dass** das nach Schritt c) erhaltene kristalline 4-Isopropyl-3-methyl-phenol einen Gehalt von mehr als 90 Gew.-%, bevorzugt von 91 bis 99 Gew.-%, besonders bevorzugt von 92 bis 99 Gew.-%, und ganz besonders bevorzugt von 92 bis 96 Gew.-% 4-Isopropyl-3-methyl-phenol aufweist.

6. Verfahren nach einem der Ansprüche 1 bis 5, **dadurch gekennzeichnet, dass** Schritt c) bei einer relativen Zentrifugalbeschleunigung von 100 bis 1000 g, bevorzugt von 200 bis 900 g, besonders bevorzugt von 600 bis 800 g, durchgeführt wird.

**Claims**

1. Method for producing 4-isopropyl-3-methylphenol from reaction mixtures obtainable by reacting meta-cresol with propene in the presence of a catalyst, **characterized in that** at least

   a) thymol and unreacted meta-cresol are largely removed from the reaction mixture by distillation, wherein a distillation residue is obtained, and
   b) from the distillation residue remaining according to step a), a suspension comprising crystalline 4-isopropyl-3-methylphenol and a liquid phase is obtained by crystallization, and
   c) from the suspension obtained according to step b), crystalline 4-isopropyl-3-methylphenol is separated from the liquid phase by the effect of centrifugal forces.

2. Method according to Claim 1 comprising the additional step of

   d) recrystallization of the crystalline 4-isopropyl-3-methylphenol obtained from step c).

3. Method according to Claim 1 or 2, **characterized in that** step c) is carried out by the effect of centrifugal forces in a centrifuge, preferably in a filter centrifuge, more preferably in a discontinuous filter centrifuge, or preferably in a horizontal centrifuge, particularly preferably in a scraper centrifuge.

4. Method according to any of Claims 1 to 3, **characterized in that** said method comprises no wash step.

5. Method according to any of Claims 1 to 4, **characterized in that** the crystalline 4-isopropyl-3-methylphenol obtained according to step c) has a 4-isopropyl-3-methylphenol content of more than 90% by weight, preferably 91 to 99% by weight, particularly preferably 92 to 99% by weight, and especially preferably 92 to 96% by weight.

6. Method according to any of Claims 1 to 5, **characterized in that** step c) is carried out at a relative centrifugal acceleration from 100 to 1000 g, preferably from 200 to 900 g, particularly preferably from 600 to 800 g.

**Revendications**

1. Procédé de fabrication de 4-isopropyl-3-méthyl-phénol à partir de mélanges réactionnels qui peuvent être obtenus par mise en réaction de méta-crésol avec du propène en présence d'un catalyseur, **caractérisé en ce qu'**au moins

   a) du thymol et du méta-crésol non réagi sont séparés en grande partie par distillation du mélange réactionnel, un résidu de distillation étant obtenu, et
   b) à partir du résidu de distillation restant selon l'étape a), une suspension contenant du 4-isopropyl-3-méthyl-phénol cristallin et une phase liquide est obtenue par cristallisation, et
   c) à partir de la suspension obtenue selon l'étape b), du 4-isopropyl-3-méthyl-phénol cristallin est séparé de la phase liquide par l'action de forces centrifuges.

2. Procédé selon la revendication 1, comprenant en outre l'étape suivante :

   d) la recristallisation du 4-isopropyl-3-méthyl-phénol cristallin obtenu à l'étape c).

3. Procédé selon la revendication 1 ou 2, **caractérisé en ce que** l'étape c) est réalisée par l'action de forces centrifuges dans une centrifugeuse, de préférence dans une centrifugeuse à filtre, de manière davantage préférée dans une centrifugeuse à filtre discontinue, ou de préférence dans une centrifugeuse horizontale, de manière particulièrement préférée dans une centrifugeuse à raclage.

4. Procédé selon l'une quelconque des revendications 1 à 3, **caractérisé en ce qu'**il ne comprend pas d'étape de lavage.

5. Procédé selon l'une quelconque des revendications 1 à 4, **caractérisé en ce que** le 4-isopropyl-3-méthyl-phénol cristallin obtenu à l'étape c) présente une teneur supérieure à 90 % en poids, de préférence de 91 à 99 % en poids, de manière particulièrement préférée de 92 à 99 % en poids, et de manière tout particulièrement préférée de 92 à 96 % en poids, en 4-isopropyl-3-méthyl-phénol.

**6.** Procédé selon l'une quelconque des revendications 1 à 5, **caractérisé en ce que** l'étape c) est réalisée à une accélération centrifuge relative de 100 à 1 000 g, de préférence de 200 à 900 g, de manière particulièrement préférée de 600 à 800 g.

**IN DER BESCHREIBUNG AUFGEFÜHRTE DOKUMENTE**

**In der Beschreibung aufgeführte Patentdokumente**

- US 3331879 A
- DE 2139622OS A
- DE 2528303OS A
- US 2603662 A
- DE 102007035515 A
- EP 2524906 A1
- DE 3824284OS A